# EUROPEAN PATENT APPLICATION

(11) **EP 4 722 714 A1**
(43) Date of publication of application: **08.04.2026**
(21) Application number: 25170776.6
(22) Date of filing: 15.04.2025
(51) Int. Cl.: G01N 30/88, G01N 33/02

(54) **SIMULTANEOUS ANALYSIS OF FOOD-BORNE DOPING SUBSTANCES USING LIQUID CHROMATOGRAPHY-MASS SPECTROMETRY (VFD: VACCINE FOR FOODBORNE DOPING)**

(30) Priority: 07.10.2024 KR 20240135481
(71) Applicant: Korea Institute of Science and Technology, Seoul 02792 (KR)
(72) Inventor: Son, Junghyun, 02792 Seoul (KR); Park, Hana, 02792 Seoul (KR)
(74) Representative: Handley, Matthew Edward

(57) **Abstract**

The present invention relates to a method for simultaneous analysis of food-borne doping substances using liquid chromatography-mass spectrometry, and more specifically, to a method for rapidly and accurately simultaneously detecting 331 doping substances using Quechers (Quick, Easy, Cheap, Effective, Rugged, and Safe) sample pretreatment method and liquid chromatography-mass spectrometry/mass spectrometry (LC-MS/MS).

The analyzing method of the present invention can be applied to a wide range of food matrices including meat, dairy products, grains and fruits, ensuring a comprehensive application range for doping control, and can rapidly and accurately detect 331 kinds of doping substances in food matrices simultaneously, which can greatly improve food safety and the efficiency of doping tests. In addition, since the process can be simplified to minimize the need for multiple testing protocols and reduce labor and operating costs, making it economically feasible, the present invention can provide solutions for various industries (food manufacturers, sports organizations, national institutions, research institutes, and universities) from agriculture to food safety and sports to quickly and accurately verify whether doping substances are present in various food matrices.

## Description

### [Technical field]

The present invention relates to a method for simultaneous analysis of food-borne doping substances using liquid chromatography-mass spectrometry, and more specifically, to a method for rapidly and accurately simultaneously detecting 331 doping substances using Quechers (Quick, Easy, Cheap, Effective, Rugged, and Safe) sample pretreatment method and liquid chromatography-mass spectrometry/mass spectrometry (LC-MS/MS).

### [Background art]

Unintentional doping by food has become a major problem for athletes, and in particular, natural substances such as higenamine contained in food, residues of animal growth promoters or pharmacological agents (e.g. clenbuterol residues in meat, meldonium residues in dairy products, etc.), and ingredients in dietary supplements can cause athletes to unintentionally violate anti-doping regulations. This problem of unintentional ingestion of prohibited substances through general food and dietary supplements poses a great challenge to athletes, sports organizations, and regulatory authorities, and to protect fair competition of athletes and the health of consumers, the concept of VFD (vaccine for foodborne doping) has been introduced, and a reliable analytical method is essential to prevent unintentional ingestion of doping substances through food and to derive accurate results from doping tests.

To this end, a rapid simultaneous screening method for multiple doping substances is being developed (Ji Hyun Lee et. al., Food Addit Contam Part A Chem Anal Control Expo Risk Assess, 37(9):1425-1436, 2020; Yonghuan Yan et. al., Microchemical Journal, 165:106142, 2021; Republic of Korea Publication No. 10-2023-0143302). However, although the current analysis technology using mass spectrometry has the sensitivity and selectivity for specific doping substances, it has limitations in screening many doping substances simultaneously, rapidly and efFiciently. In particular, there is a lack of a high-throughput analysis method that can rapidly process various doping substances in foods.

Each food matrix has unique chemical properties, and a customized pretreatment process is required to properly analyze it. However, these individual sample pretreatment processes are often complex and time-consuming, which lengthens the overall analysis process and can be a major obstacle in situations where rapid screening is required.

In addition, simultaneous detection of more than 300 doping substances requires increased analysis time for each substance, which significantly increases the overall analysis time, while conventional analysis methods require analysis of each substance separately, which increases the overall analysis time for a single sample. Furthermore, some techniques are only applicable to certain types of samples and are difficult to apply to a wide range of food samples.

### [Disclosure]

### [Technical Problem]

Accordingly, the present invention has made great efforts to develop an analytical method applicable to various food matrices and capable of simultaneously detecting more than 300 doping substances in a quick and easy manner. As a result, the present invention confirms that using Quechers (Quick, Easy, Cheap, Effective, Rugged, and Safe) sample pretreatment method and liquid chromatography-mass spectrometry/mass spectrometry (LC-MS/MS), 331 doping substances can be rapidly and accurately detected simultaneously.

Therefore, the purpose of the present invention is to provide a method for simultaneous analysis of food-borne doping substances using the Quechers sample pretreatment method and liquid chromatography-mass spectrometry/mass spectrometry (LC-MS/MS).

### [Technical solution]

To achieve the above-mentioned purpose, the present invention provides a method for simultaneous analysis of food-borne doping substances comprises the steps of f:
(a) homogenizing food samples and then preparing an analytical sample by mixing it with water and acetonitrile;
(b) pretreating the analytical sample with a salt mixture comprising MgSO₄, NaCl, Na₃C₆H₅O₇, and C₆H₆Na₂O₇-1.5H₂O;
(c) purifying the pretreated analytical sample by QuEChERS dispersive solid phase extraction method (dSPE) using MgSO₄, C18EC column, graphitized carbon black (GCB) and Primary Secondary Amine (PSA; N-propyl ethylene diamine based SPE column);
(d) concentrating the analytical sample with a nitrogen concentrator and then redissolving the analytical sample in a 0.1% (v/v) formic acid aqueous solution; and
(e) centrifuging the redissolved analytical sample and then analyzing the supernatant by liquid chromatography-mass spectrometry (LC-MS/MS) for simultaneous analysis of food-borne doping substances.

In a preferred embodiment of the present invention, the liquid chromatography-mass spectrometry of step (e) above may use a Kinetex 2.6-µm C18 column and a Security Guard ULTRA Cartridges column.

In another preferred embodiment of the present invention, the liquid chromatography-mass spectrometry of step (e) above can be performed using water containing 0.1% (v/v) formic acid (solution A) and 0.1% (v/v) formic acid (B solution) as mobile phases, eluting with 2% (v/v) B solution (0 to 0.5 min), 2% (v/v) to 95% (v/v) B solution (0.5 to 8.5 min), 95% (v/v) B solution (8.5 to 9 min), 95% (v/v) to 2% (v/v) B solution (9 to 9.01 min), and 2% (v/v) B solution (9.01 to 10 min).

In another preferred embodiment of the present invention, the mobile phase can be injected into liquid chromatography at a rate of 0.5 mL/min.

In another preferred embodiment of the present invention, the food product may be an agricultural product, livestock product, natural spice, dairy product, processed animal product, edible oil, beverage, processed food, ready-to-eat food, snack, or dietary.

In another preferred embodiment of the present invention, the doping substances simultaneously detected by the method can be at least one selected from the group consisting of: Efaproxiral (RSR-13), Acebutolol, Alprenolol, Atenolol, Befunolol, Betaxolol, Bisoprolol, Bufuralol, Bupranolol, Carteolol, Carvedilol, Carvedilol(5'-Hydroxyphenyl Carvedilol), Carvedilol(Desmethylcarvedilol), Celiprolol, Esmolol, Esmolol acid, Labetalol, Levobunolol, Mepindolol, Metipranolol, Metoprolol, Nadolol, Nebivolol, Oxprenolol, Penbutolol, Pindolol, Propranolol, Propranolol(4-OH-propranolol), Sotalol, Timolol, Formebolone_M, Gestrinone, Gestrinone (6a-OH-Gestrinone), Methyldienolone, Methylribolone, Norclostebol, Oxandrolone, Oxandrolone(Epioxandrolone), Prostanozol (3'-OH-Prostanozol), Stanozolol, Stanozolol(3'-OH-stanozolol)], Tetrahydrogestrinone, Andarine, Andarine(O-Dephenylandarine), Andarine(Other_OH-Flutamide), Clenbuterol, LGD-4033, Ostarine, Ostarine(Bicalutamide), Ostarine(O-Dephenylostarine), Ractopamine, RAD-140, Zeranol(other_Zearalanone), Zeranol(other_Zearalenone), Zilpaterol, FG-2216(HIF-prolyl hydroxylase inhibitor), FG-4592(Prolyl hydroxylase inhibitor), Molidustat (Bay85-3934), Leuprorelin, LHRH(1-3)-OH([luteinizing hormone(LH)-releasing hormone (1-3)-OH], Nafarelin, Triptorelin, Alexamorelin (3-6), Alexamorelin (3-6)-OH, Anamorelin, AOD-9604(7-16), GHRP(growth hormone-releasing peptide)-1 (2-4)-OH, GHRP-1(3-6), GHRP-1(3-7), GHRP-2(1-3)-OH, GHRP-2(1-6), GHRP-4, GHRP-4(1-3), GHRP-5, GHRP-5(1-3), GHRP-5(1-4), GHRP-6(2-5)-OH, GHRP-6(2-6)-OH, Hexarelin (1-3)-OH, Ibutamoren, Ipamorelin (1-4)-OH, Tabimorelin, TB-500(1-2)-OH[Thymosin Beta-4(1-2)-OH], TB-500(17-23), Alarelin, Fertirelin, Perforelin, Bambuterol, Bambuterol(Terbutaline), Fenoterol, Higenamine, Higenamine (Coclaurine), Indcaterol, Olodaterol, Procaterol, Reproterol, Ritodrine, Salmeterol, Tretoquinol, Tulobuterol, Vilanterol, T.Formoterol, T.Salbutamol, Aminoglutethimide, Anastrozole, Androstatrienedione (ATD), Exemestane, Exemestane(17-hydroexemestane), Letrozole, Testolactone, Testolactone M, Bazedoxifene, Clomiphene(4-OH-Clomiphene_M), Fulvestrant, Raloxifene, Tamoxifen, Tamoxifen(3-Hydroxy-4-methoxytamoxifen), Toremifene, Toremifene(4-Hydroxy-Toremifene), GW1516-Sulfone, GW1516-Sulfoxide, Meldonium, SR9009, SR9009(M2), SR9009(M6), Trimetazidine, Trimetazidine(Lomerizine_M6), Trimetazidine(Other_Lomerizine), 4-amino-6-chloro-1,3-benzenedisulphonamide (ACB), Acetazolamide, Althiazide, Amiloride, Bendroflumethiazide, Benzthiazide, Benzylhydrochlorothiazide, Brinzolamide, Bumetanide, Butizide, Chlorazanil, Chlorazanil(Other Cycloguanil), Chlorazanil(Other_Proguanil), Chlorothalidone, Chlorothiazide, Clopamide, Conivaptan, Cyclopenthiazide, Cyclothiazide, Dorzolamide, Epitizide, Eplerenone, Eplerenone(6b-OH-eplerenone), Ethacrynic acid, Furosemide, Hydrochlorothiazide, Hydroflumethiazide, Indapamide, Lixivaptan, Mefruside, Mefruside(5-oxo-mefruside), Methazolamide, Methylclothiazide, Metolazone, Mozavaptan, Piretanide, Polythiazide, Quinethazone, Spironolactone, Spironolactone(Canrenoicacid), Spironolactone(Canrenone), Tolvaptan, Torasemide, Triamterene, Trichloromethiazide, Xipamide, ATFB, M Desmopressin, M Desmopressin(aa1-7], M Desmopressin(aa1-7)-OH, M Felipressin, M Probenecid, P HES(hydroxyethyl starche), Adrafinil/Modafinil, Amfepramone, Amfetamine, Amiphenazole, Benfluorex, Benfluorex_M1, Benfluorex_M2, Benzylpiperazine, Bromantane(6-OH-bromantane), Clobenzorex, Cocaine, Cocaine (Benzoylecgonine), Cocaine (Methylecgonine), Cropropamide, Crotetamide, Fencamine, Fenetylline, Fenfluramine, Fenproporex, Fonturacetam, Furfenorex, Mefenorex, Mephentermine (b-OH-mephentermine), Mesocarb, Mesocarb (p-OH-Mesocarb), Methamphetamine, Modafinil, Modafinil+Na, Modarinil(Modafinil acid_p), Norfenfluramine, p-Methylamphetamine (iii), Phendimetrazine, Phentermine (iii), Phentermine (Oxethazaine), Prenylamine, Prolintane, 3,4-Methylenedioxyethylamfetamine (MDEA), 4-Fluoroamphetamine& 2-FA), 5-methylhexan-2-amine, b-Methylphenylethylamine, Benzphetamine, Cathinone, Cathinone (Mephedrone), Cathinone (Methedrone), Cyclazodone, Dimethylamphetamine, DMA(iii), 3,4-Dimethoxyphenethylamine (DMPEA), Dobutamine, Etamivan, Etilamfetamine (iii), Famprofazone, Fenbutrazate, Fencamfamin, Heptaminol, Isometheptene, Meclofenoxate(4-CPA), Methoxyphenamine, Methylenedioxyamphetamine (MDA), Methylenedioxymethamphetamine (MDMA), Methylhexanamine, Methylphenidate, Methylphenidate (Ritalinic acid), Morazone, N-Ehtyl-phenyl-2-butanamine, Norfenefrine, Octopamine, Orteamine (iii), Oxilofrine, p-OH-amphetamine (Other_Mebeverine), p-OH-amphetamine (Other_PMEA)], Pemoline, Pentetrazol, Phenmetrazine, Phenpromethamine, Pholedrine, Propylhexedrine, Selegiline, Selegiline M, Strychinine, Tuaminoheptane, T.Ephedrine, T.Methylephedrine, T.Pseudoephedrine, Buprenorphine, Buprenorphine(Norbuprenorphine), Dextromoramide, Dihydrocodeine, Fentanyl, Fentanyl(Norfentanyloxalate), Heroin, Hydromorphone, Mefentanyl, Methadone, Methadone(EDDP), Morphine(Ethylmorphine), Oxycodone, Oxymorphone, Pentazocine, Pethidine, Pethidine(Norpethidine), Sufentanil, T.Morphine, COOH-JWH-018, COOH-JWH-073, OH-JWH-018, OH-JWH-073, 6a-Methylprednisolone, Beclomethasone, Betamethasone, Budesonide, Budesonide(6-OH-Budesonide], Ciclesonide(des-ciclesonide), Cortisol, Cortisone, Deflazacort, Deflazacort(21-desacetyldeflazacort), Desonide, Desoximethasone, Fludrocortisone, Flumethasone, Flunisolide, Fluocortolone, Fluorometholone, Fluticason furoate, Fluticason(propionatecarboxymetabolite), Mometasone, Prednisolone, Prednisolone(20b-OH-prednisolone), Prednisone, Prednisone(20b-OH-prednisone), Triamcinolone, Triamcinolone acetonide, Triamcinolone acetonide(6b-OH-TA)], 4-OH-Dutasteride, 6-OH-Dutasteride, Finasteride (Carboxyfinasteride), Fluconazole, Ketoconazole, Miconazole, Bupropion, Caffeine, Nicotine, Phenylephrine, Pipradol, Synephrine, Hydrocodone, Hydrocodone(Norhydrocodone), and Tramadol.

### [Advantageous Effects]

In the present invention, the method for simultaneous analysis of food-borne doping substances using the Quechers sample pretreatment method and liquid chromatography-mass spectrometry/mass spectrometry (LC-MS/MS) has been established. The analyzing method of the present invention can be applied to a wide range of food matrices including meat, dairy products, grains, and fruits, thereby ensuring a comprehensive application range for doping management, and can rapidly and accurately detect 331 kinds of doping substances in food matrices simultaneously, thereby greatly improving food safety and the efficiency of doping tests.

In addition, since the process can be streamlined to minimize the need for multiple testing protocols and is economically viable by reducing labor and operating costs, the present invention can provide a solution for a variety of industries (food manufacturers, sports organizations, national institutions, research institutes and universities) ranging from agriculture to food safety to sports to quickly and accurately verify the presence of doping substances in a variety of food matrices.

### [Description of Drawing]

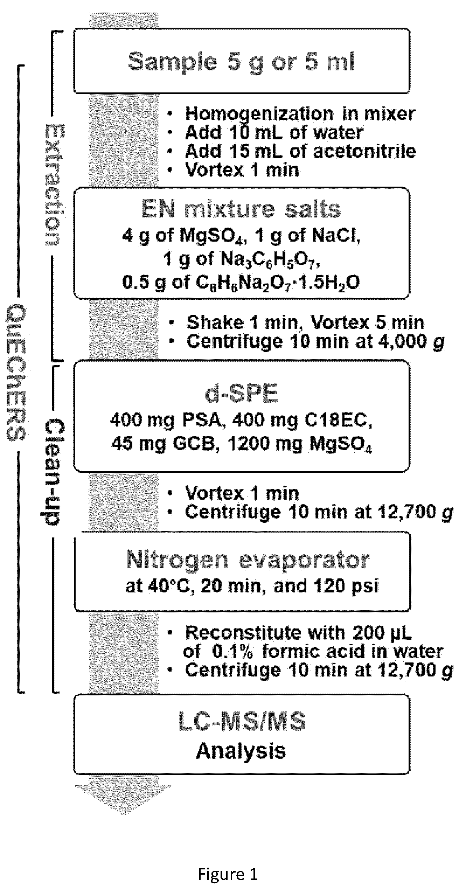

Figure 1 is a schematic diagram illustrating the present single protocol that can effectively preprocess various food matrices.

### [Mode of the Invention]

Hereinafter, the present invention will be described in detail.

The present invention relates to a method for simultaneous analysis of food-borne doping substances comprises the steps of:
(a) homogenizing food samples and then preparing an analytical sample by mixing it with water and acetonitrile;
(b) pretreating the analytical sample with a salt mixture comprising MgSO₄, NaCl, Na₃C₆H₅O₇, and C₆H₆Na₂O₇-1.5H₂O;
(c) purifying the pretreated analytical sample by QuEChERS dispersive solid phase extraction method (dSPE) using MgSO₄, C18EC column, graphitized carbon black (GCB) and Primary Secondary Amine (PSA; N-propyl ethylene diamine based SPE column);
(d) concentrating the analytical sample with a nitrogen concentrator and then redissolving the analytical sample in a 0.1% (v/v) formic acid aqueous solution; and
(e) centrifuging the redissolved analytical sample and then analyzing the supernatant by liquid chromatography-mass spectrometry (LC-MS/MS) for simultaneous analysis of food-borne doping substances.

In order to develop an analytical method applicable to various food matrices and capable of simultaneously detecting more than 300 doping substances quickly and easily, in the present invention, a simultaneous analysis method of food-borne doping substances using Quechers sample pretreatment method and liquid chromatography-mass spectrometry/mass spectrometry (LC-MS/MS) were established.

The analyzing method of the present invention is a simultaneous analysis method for doping substances derived from various foods, which can rapidly and accurately detect 331 doping substances simultaneously in food matrix, and can detect a wider range of substances than existing analysis methods. In addition, in the present invention, the processing time can be significantly shortened compared to existing individual analysis methods, and multiple substances can be detected in a single analysis, thereby reducing laboratory operating costs and increasing economic efficiency.

The analyzing method of the present invention has universal applicability to various food matrices, and has the advantage of being effectively applicable to various food matrices such as meat, dairy products, grains, and fruits with a single standardized method.

In addition, the analyzing method of the present invention simplifies the complex preprocessing process, thereby increasing the efficiency of the entire analysis process, and since it enables simultaneous analysis of other compounds in addition to detection of doping substances, it can be applied to various fields such as food safety testing, environmental analysis, and drug detection.

The specific analyzing method of the present invention is as follows (Fig. 1).

### Sample Pretreatment Step

Step 1: homogenizing food sample and then preparing an analytical sample by mixing it with water and acetonitrile.

Step 2: Pretreating the analytical sample with a salt mixture comprising MgSO₄, NaCl, Na₃C₆H₅O₇, C₆H₆Na₂O₇-1.5H₂O. The analytical sample pretreated with the salt mixture was homogenized and centrifuged to obtain a supernatant.

In the pretreatment process of the present invention, Na₃C₆H₅O₇, and C₆H₆Na₂O₇-1.5H₂O were used to ensure the stability of the doping substances within each food, maintain the pH, and enable efficient extraction.

### Sample Purification Step

Step 3: The pretreated analytical samples were purified by QuEChERS dispersive solid phase extraction method (dSPE) using MgSO₄, C18EC column, graphite-carbon black (GCB) and ethylene diamine-n-propyl (PSA).

In the pretreatment process, Na₃C₆H₅O₇, and C₆H₆Na₂O₇-1.5H₂O were used to ensure the stability of doping substances in each food, maintain the pH, and enable efficient extraction. In the sample purification process, C18EC, PSA, and GCB components can be utilized together to effectively handle various types of impurities from various food matrices at once, and in particular, complex organic matter including polar organic acids, sugars, carotenoids, chlorophyll, lipids, and fats can be adsorbed to increase sample purification efficiency.

In addition, in the present invention, a combination of a salt mixture and a QuEChERS dispersive solid-phase extraction (dSPE) method in the sample pretreatment process is established as a single protocol, and consistent pretreatment and efficiency can be maintained even when the same combination is used in various food matrices (e.g., fruits, vegetables, grains, meat, dairy products, etc.).

### Concentration and Reconstitution Step

Step 4: The purified sample was concentrated with a nitrogen concentrator and then re-dissolved in a 0.1% (v/v) aqueous solution of formic acid.

The concentration conditions of re-dissolving in 200 µL of a 0.1% aqueous formic acid solution play an important role in the final stage of the analysis, and the conditions of the present invention can contribute to increasing the stability and detection limit of the compounds to be analyzed.

### LC-MS/MS Analysis Step

Step 5: The above re-dissolved analytical sample was centrifuged, and the supernatant was simultaneously analyzed for food-borne doping substances using liquid chromatography-mass spectrometry (LC-MS/MS).

The column used in the present invention may be a nonpolar column, which can effectively separate various organic compounds, and by using a guard column, the column life can be extended, and damage caused by contaminants can be prevented, thereby increasing the reliability and reproducibility of the analysis. Preferably, a Kinetex 2.6-µm C18 column and a Security Guard ULTRA Cartridges column were used in the present invention. Using dual-mode ESI, about 300 types of doping substances can be detected at once through analysis.

The mobile phase was a mixed solvent system capable of dissolving both polar and nonpolar components. Water with 0.1% (v/v) formic acid (solution A) and methanol with 0.1% (v/v) formic acid (solution B) were used as the mobile phases. In order to optimize the separation efficiency of each component by controlling the ratio of the mobile phase over time, the gradient elution conditions were 2% (v/v) B solution (0 to 0.5 min), 2% (v/v) ~ 95% (v/v) B solution (0.5 to 8.5 min), 95% (v/v) B solution (8.5 to 9 min), 95% (v/v) ~ 2% (v/v) B solution (9 to 9.01 min), and 2% (v/v) B solution (9.01 to 10 min).

The gradient setup, starting from a 2% B (methanol) solution and rapidly increasing to 95% B solution over 8.5 minutes, and then rapidly returning to 2% B solution over 9 minutes, was optimized to dramatically reduce analysis time while maintaining high separation efficiency. The ability to achieve fast and efficient separations in such a short time is particularly important for high-throughput applications, such as the simultaneous screening of over 300 doping substances in less than 10 minutes.

The analysis time can be shortened by providing optimal separation conditions through flow rate control, and in the present invention, the mobile phase was injected into the liquid chromatography at a rate of 0.5 mL/min.

In the preset process of detecting doping substances, the mass spectrometer ion mode used ESI (Electrospray Ionization), and the optimal precursor ion and product ion pairs for each doping substances were selected, and the appropriate collision energy was set for each pair to secure maximum sensitivity. The oscilloscope mode applied MRM (Multiple Reaction Monitoring) to select and detect specific ions, and analyze them with high selectivity.

In addition, the present invention optimizes the ionization conditions as follows to increase the detection sensitivity of the sample components: Ion spray voltage = (+) 4000 V, (-) 3500 V, sheath gas = 60 Arb, Aux gas = 15 Arb, Sweep gas = 1 Arb, Ion transfer tube temperature = 325°C, Vaporization temp. = 350°C.

In the present invention, the food product may be an agricultural product, livestock product, natural spice, dairy product, processed animal product, edible oil, beverage, processed food, ready-to-eat food, snack, or dietary, and more particularly may be any of the food products listed in Tables 1 to 3 below.

In the present invention, the doping substances simultaneously detected by the above method can be at least one selected from the group consisting of: Efaproxiral (RSR-13), Acebutolol, Alprenolol, Atenolol, Befunolol, Betaxolol, Bisoprolol, Bufuralol, Bupranolol, Carteolol, Carvedilol, Carvedilol(5'-Hydroxyphenyl Carvedilol), Carvedilol(Desmethylcarvedilol), Celiprolol, Esmolol, Esmolol acid, Labetalol, Levobunolol, Mepindolol, Metipranolol, Metoprolol, Nadolol, Nebivolol, Oxprenolol, Penbutolol, Pindolol, Propranolol, Propranolol(4-OH-propranolol), Sotalol, Timolol, Formebolone_M, Gestrinone, Gestrinone (6a-OH-Gestrinone), Methyldienolone, Methylribolone, Norclostebol, Oxandrolone, Oxandrolone(Epioxandrolone), Prostanozol (3'-OH-Prostanozol), Stanozolol, Stanozolol(3'-OH-stanozolol)], Tetrahydrogestrinone, Andarine, Andarine(O-Dephenylandarine), Andarine(Other_OH-Flutamide), Clenbuterol, LGD-4033, Ostarine, Ostarine(Bicalutamide), Ostarine(O-Dephenylostarine), Ractopamine, RAD-140, Zeranol(other_Zearalanone), Zeranol(other_Zearalenone), Zilpaterol, FG-2216(HIF-prolyl hydroxylase inhibitor), FG-4592(Prolyl hydroxylase inhibitor), Molidustat (Bay85-3934), Leuprorelin, LHRH(1-3)-OH([luteinizing hormone(LH)-releasing hormone (1-3)-OH], Nafarelin, Triptorelin, Alexamorelin (3-6), Alexamorelin (3-6)-OH, Anamorelin, AOD-9604(7-16), GHRP(growth hormone-releasing peptide)-1 (2-4)-OH, GHRP-1(3-6), GHRP-1(3-7), GHRP-2(1-3)-OH, GHRP-2(1-6), GHRP-4, GHRP-4(1-3), GHRP-5, GHRP-5(1-3), GHRP-5(1-4), GHRP-6(2-5)-OH, GHRP-6(2-6)-OH, Hexarelin (1-3)-OH, Ibutamoren, Ipamorelin (1-4)-OH, Tabimorelin, TB-500(1-2)-OH[Thymosin Beta-4(1-2)-OH], TB-500(17-23), Alarelin, Fertirelin, Perforelin, Bambuterol, Bambuterol(Terbutaline), Fenoterol, Higenamine, Higenamine (Coclaurine), Indcaterol, Olodaterol, Procaterol, Reproterol, Ritodrine, Salmeterol, Tretoquinol, Tulobuterol, Vilanterol, T.Formoterol, T.Salbutamol, Aminoglutethimide, Anastrozole, Androstatrienedione (ATD), Exemestane, Exemestane(17-hydroexemestane), Letrozole, Testolactone, Testolactone M, Bazedoxifene, Clomiphene(4-OH-Clomiphene_M), Fulvestrant, Raloxifene, Tamoxifen, Tamoxifen(3-Hydroxy-4-methoxytamoxifen), Toremifene, Toremifene(4-Hydroxy-Toremifene), GW1516-Sulfone, GW1516-Sulfoxide, Meldonium, SR9009, SR9009(M2), SR9009(M6), Trimetazidine, Trimetazidine(Lomerizine_M6), Trimetazidine(Other_Lomerizine), 4-amino-6-chloro-1,3-benzenedisulphonamide (ACB), Acetazolamide, Althiazide, Amiloride, Bendroflumethiazide, Benzthiazide, Benzylhydrochlorothiazide, Brinzolamide, Bumetanide, Butizide, Chlorazanil, Chlorazanil(Other Cycloguanil), Chlorazanil(Other_Proguanil), Chlorothalidone, Chlorothiazide, Clopamide, Conivaptan, Cyclopenthiazide, Cyclothiazide, Dorzolamide, Epitizide, Eplerenone, Eplerenone(6b-OH-eplerenone), Ethacrynic acid, Furosemide, Hydrochlorothiazide, Hydroflumethiazide, Indapamide, Lixivaptan, Mefruside, Mefruside(5-oxo-mefruside), Methazolamide, Methylclothiazide, Metolazone, Mozavaptan, Piretanide, Polythiazide, Quinethazone, Spironolactone, Spironolactone(Canrenoicacid), Spironolactone(Canrenone), Tolvaptan, Torasemide, Triamterene, Trichloromethiazide, Xipamide, ATFB, M Desmopressin, M Desmopressin(aa1-7], M Desmopressin(aal-7)-OH, M Felipressin, M Probenecid, P HES(hydroxyethyl starche), Adrafinil/Modafinil, Amfepramone, Amfetamine, Amiphenazole, Benfluorex, Benfluorex_M1, Benfluorex_M2, Benzylpiperazine, Bromantane(6-OH-bromantane), Clobenzorex, Cocaine, Cocaine (Benzoylecgonine), Cocaine (Methylecgonine), Cropropamide, Crotetamide, Fencamine, Fenetylline, Fenfluramine, Fenproporex, Fonturacetam, Furfenorex, Mefenorex, Mephentermine (b-OH-mephentermine), Mesocarb, Mesocarb (p-OH-Mesocarb), Methamphetamine, Modafinil, Modafinil+Na, Modarinil(Modafinil acid_p), Norfenfluramine, p-Methylamphetamine (iii), Phendimetrazine, Phentermine (iii), Phentermine (Oxethazaine), Prenylamine, Prolintane, 3,4-Methylenedioxyethylamfetamine (MDEA), 4-Fluoroamphetamine& 2-FA), 5-methylhexan-2-amine, b-Methylphenylethylamine, Benzphetamine, Cathinone, Cathinone (Mephedrone), Cathinone (Methedrone), Cyclazodone, Dimethylamphetamine, DMA(iii), 3,4-Dimethoxyphenethylamine (DMPEA), Dobutamine, Etamivan, Etilamfetamine (iii), Famprofazone, Fenbutrazate, Fencamfamin, Heptaminol, Isometheptene, Meclofenoxate(4-CPA), Methoxyphenamine, Methylenedioxyamphetamine (MDA), Methylenedioxymethamphetamine (MDMA), Methylhexanamine, Methylphenidate, Methylphenidate (Ritalinic acid), Morazone, N-Ehtyl-phenyl-2-butanamine, Norfenefrine, Octopamine, Orteamine (iii), Oxilofrine, p-OH-amphetamine (Other_Mebeverine), p-OH-amphetamine (Other_PMEA)], Pemoline, Pentetrazol, Phenmetrazine, Phenpromethamine, Pholedrine, Propylhexedrine, Selegiline, Selegiline M, Strychinine, Tuaminoheptane, T.Ephedrine, T.Methylephedrine, T.Pseudoephedrine, Buprenorphine, Buprenorphine(Norbuprenorphine), Dextromoramide, Dihydrocodeine, Fentanyl, Fentanyl(Norfentanyloxalate), Heroin, Hydromorphone, Mefentanyl, Methadone, Methadone(EDDP), Morphine(Ethylmorphine), Oxycodone, Oxymorphone, Pentazocine, Pethidine, Pethidine(Norpethidine), Sufentanil, T.Morphine, COOH-JWH-018, COOH-JWH-073, OH-JWH-018, OH-JWH-073, 6a-Methylprednisolone, Beclomethasone, Betamethasone, Budesonide, Budesonide(6-OH-Budesonide], Ciclesonide(des-ciclesonide), Cortisol, Cortisone, Deflazacort, Deflazacort(21-desacetyldeflazacort), Desonide, Desoximethasone, Fludrocortisone, Flumethasone, Flunisolide, Fluocortolone, Fluorometholone, Fluticason furoate, Fluticason(propionatecarboxymetabolite), Mometasone, Prednisolone, Prednisolone(20b-OH-prednisolone), Prednisone, Prednisone(20b-OH-prednisone), Triamcinolone, Triamcinolone acetonide, Triamcinolone acetonide(6b-OH-TA)], 4-OH-Dutasteride, 6-OH-Dutasteride, Finasteride (Carboxyfinasteride), Fluconazole, Ketoconazole, Miconazole, Bupropion, Caffeine, Nicotine, Phenylephrine, Pipradol, Synephrine, Hydrocodone, Hydrocodone(Norhydrocodone), and Tramadol.

**[Table 1] List of commercial food and dietary supplements analyzed using the present rapid analyzing method (1)**

| | | |
|---|---|---|
| Natural products | Agricultural products | Beet |
| | | Brazil nut |
| | | Cinammon |
| | | Hampseed |
| | | Licorice |
| | | Lotus seed |
| | | Nut |
| | | Oat |
| | | Yam |
| | Livestock products | Badger Gamtu |
| | | Badger Gamtu2 |
| | | Beef |
| | | Beef Blood |
| | | Beef Lungs |
| | | Chicken |
| | | Chicken Cartilage |
| | | Chicken Coop |
| | | Chicken Livers |
| | | Egg |
| | | Pig Heartache |
| | | Pig Liver |
| | | Pig Lung |
| | | Pig Testicles |
| | | Pig Tongue |
| | | Pork |

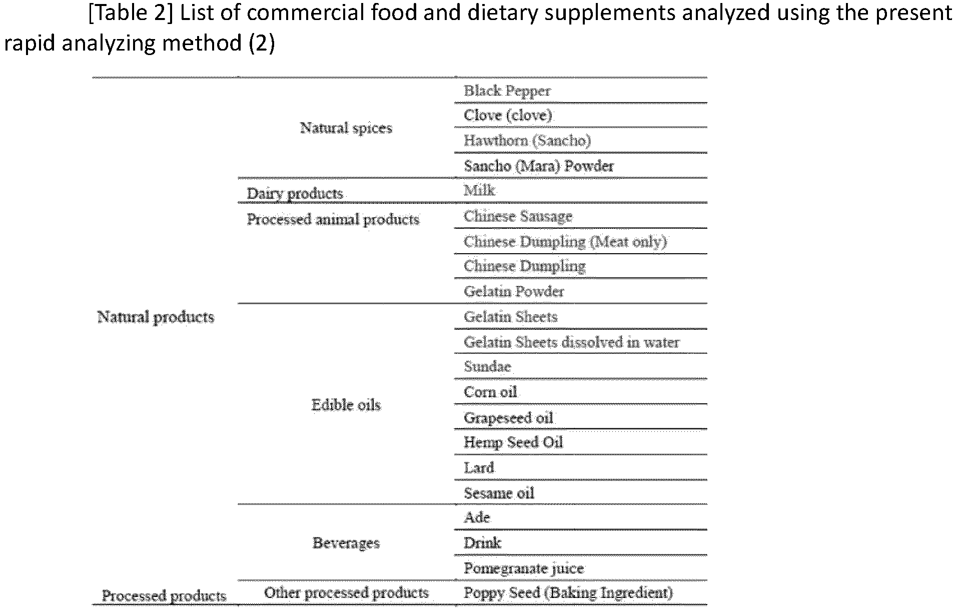

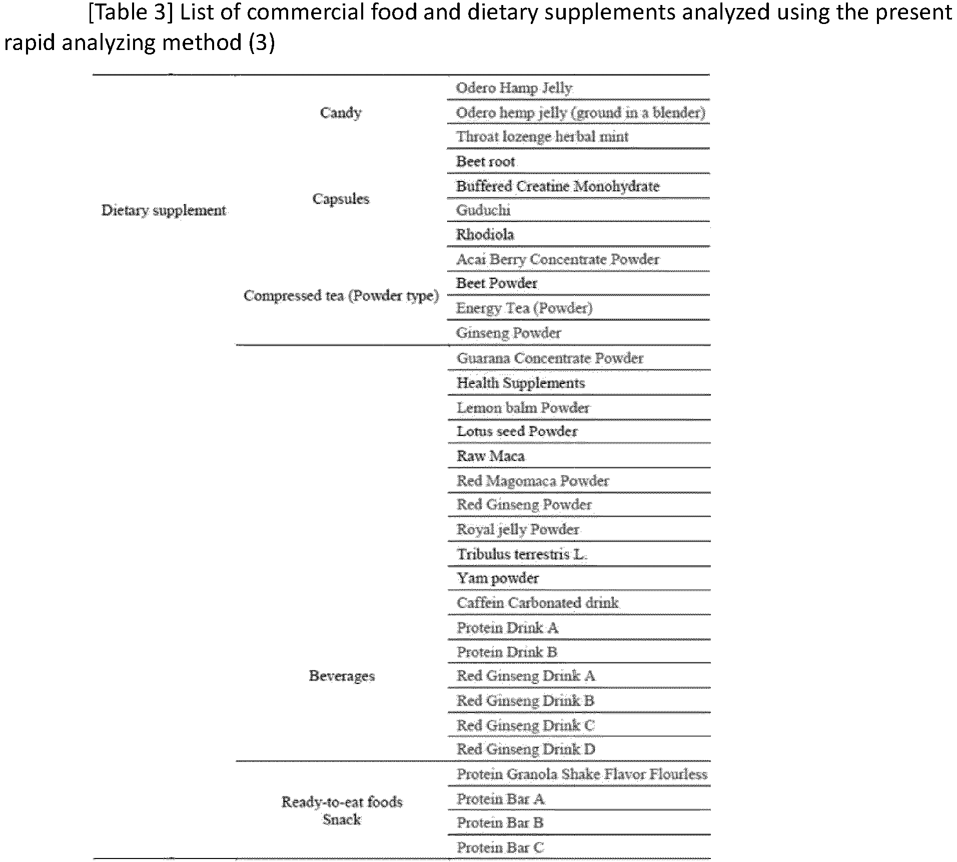

Tables 1 to 3 above indicate that the analytical method of the present invention is not limited to a specific food or dietary supplement, but can be applied across a variety of matrices, including agricultural products, meat products, processed foods, dietary supplements, and the like, and the analytical method of the present invention is designed to rapidly doping substances with different chemical properties in a variety of food, which can increase the of the analytical technique and from existing

In a specific embodiment of the present invention, it was confirmed that 331 types of doping substances can be simultaneously detected in the food matrix quickly and accurately using the present simultaneous analysis method of food-borne doping substances using Quechers sample pretreatment method and liquid chromatography-mass spectrometry/mass spectrometry (LC-MS/MS) (Tables 6 to 19).

The analyzing method of the present invention can accurately identify substances through an optimized extraction process, which can provide reliable results by reducing the risk of false positives or false negatives, which can greatly improve the reliability of doping control and prevent penalties due to false positives. In addition, the combination of QuEChERs extraction and LC-MS/MS improves the sensitivity and selectivity of the analytical method, allowing for the detection of even trace amounts of prohibited substances.

Furthermore, the analyzing method of the present invention has high sensitivity and selectivity, which can provide a robust monitoring system that can adapt to evolving doping strategies to ensure the long-term effectiveness of doping control.

Thus, the analyzing method of the present invention can be effectively utilized in the verification and monitoring of food supplied to athletes' camps and training facilities, as well as in the quality assurance and inspection of the functional food and dietary supplement industry.

The present invention will now be described in more detail with reference to the following examples.

These embodiments are intended solely to illustrate the invention, and it will be apparent to one of ordinary skill in the art that the scope of the invention is not to be construed as limited by these embodiments.

### [Example 1] Quechers Sample Pretreatment and Purification

This example provided a method for sample pretreatment and purification using the Quechers (Quick, Easy, Cheap, Effective, Rugged, and Safe) method for the analysis of doping substances in various food samples. The method was simple, efficient, and applicable to a variety of food matrices. The specific pretreatment and purification process of the present invention was shown in FIG. 1.

### 1. Type of sample

The present invention can analyze a wide variety of food groups. Specifically, agricultural products, livestock products, processed foods, dietary supplements, etc.
* Agricultural products include beets, Brazil nuts, cinnamon, hemp seeds, licorice, lotus seeds, oats, hemp, etc.
* Livestock products include a variety of livestock products such as beef, bovine blood, bovine lungs, chicken, pork liver, pork lungs, pork tongue, pork testicles, etc.
* Processed foods include processed spices and edible oils, such as commercial pepper, clove, sansho powder, gelatin powder, grape seed oil, sesame oil, corn oil, etc.
* Beverages include a variety of beverage products, including pomegranate juice, energy drinks, protein drinks, red ginseng drinks, etc.
* Dietary supplements include beetroot, guarana concentrate, red ginseng powder, royal jelly powder, wild maca, and various other supplements can be analyzed

In addition, processed animal products (e.g., Chinese sausages, dumplings), baking ingredients (poppy seeds), snack foods (protein bars), etc. can also be by the Quechers pretreatment and purification method of the present invention.

### 2. Experiment method

(1) Sample pretreatment
   After homogenizing the food sample to be analyzed using a blender or similar device, it was accurately weighed at 5 g or 5 mL and placed in a 50 mL centrifuge tube.
(2) Addition of extraction solvent
   * 10 mL of water and 15 mL of acetonitrile (ACN) were added.
   * Mixing by vortexing.
(3) Addition of extraction salt mixture
   * EN mixture salts were added to the extraction mixture:
   * 4 g of MgSO₄ (magnesium sulfate anhydrous),
   * 1 g of NaCl (sodium chloride),
   * 1g of Na₃C₆H₅O₇ (trisodium citrate),
   * 0.5 g of C₆H₈Na₂O₇·1.5H₂O (citric acid dihydrate).
   * To increase the extraction efficiency, the mixture was shaken for one minute using a ceramic homogenizer to promote extraction. After vortexing, the mixture was centrifuged at 4°C for 10 minutes at 4,000g.
(4) Extraction and purification of the supernatant
   * After centrifugation, 8 mL of the supernatant was taken and transferred to the dispersion purification (d-SPE) kit. At this time, the d-SPE kit contained MgSO₄, C18EC, PSA (Primary Secondary Amine), and GCB (Graphitized Carbon Black) components for sample purification and removal of interfering substances.
   * A ceramic homogenizer was used to ensure that the supernatant and salts were mixed evenly, thereby increasing the purification efficiency. After mixing, the mixture was vortexed and centrifuged at 4°C for 10 minutes at 12,700g.
(5) Concentration and reconstitution
   * 2 mL of the supernatant was transferred to a glass tube, and the solvent was evaporated under the conditions of 40°C, 20 minutes, and 120 psi using a nitrogen concentrator.
   * After the solvent was evaporated, it was reconstituted with 200 µL of 0.1% formic acid solution and vortexed again.
(6) Preparation for final analysis
   * After transferring the reconstituted sample to an e-tube, centrifuge at 12,000 rpm for 10 minutes.
   * Only the supernatant was taken and placed in an LC vial, and doping substances were analyzed using LC-MS/MS.

**[Table 4] Components and roles of the sample pretreatment process**

| **Step** | **Used Kit and components thereof** | **Role of components** |
|---|---|---|
| **Extraction** | Agilent QuEChERS kit 5982-5650CH | Extraction of doping substances from the sample |
| | 4 g MgSO₄ | Removal of moisture to dry the sample and increase the solubility of the sample during the extraction process |
| | 1 g NaCl | Increase the ionic strength of the solvent to increase the extraction efficiency of doping substances |
| | 1 g Na₃C₆H₅O₇ | Acts as a pH regulator to ensure the stability of doping substances and optimize chemical reactions during the extraction process |
| | 0.5 g C₆H₆Na₂O₇-1.5H₂O | Acts as additional pH regulator and increases extraction efficiency of doping substances |
| **Purification** | Agilent QuEChERS kit 5982-0029CH | Purification of extracted substances |
| | 1200 mg MgSO₄ | Helps remove additional moisture and dry the sample |
| | 400 mg C18EC | Adsorb and remove non-polar substances and help purify doping substances to be analyzed |
| | 400 mg PSA | Removes saccharides and organic acids and purifies impurities in the sample |
| | 45 mg GCB | Adsorb pigments and other complex organic substances to increase the purification efficiency of the sample |

### [Example 2] Simultaneous analysis of doping substances using liquid chromatography-mass spectrometry/mass spectrometry

Liquid chromatography-mass spectrometry (LC-MS/MS) was performed using the sample purified in < Example 1> above, and the specific experimental conditions were shown in Table 5 below.

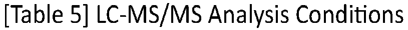

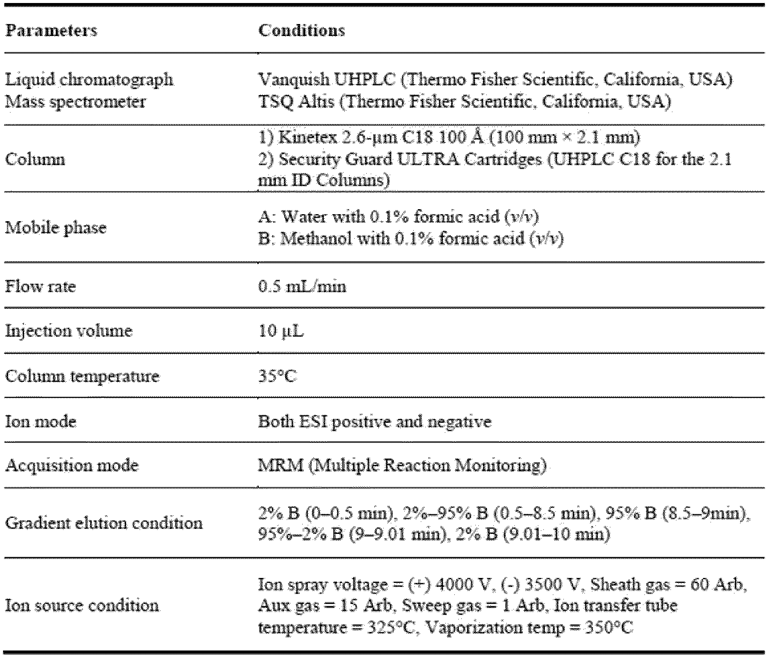

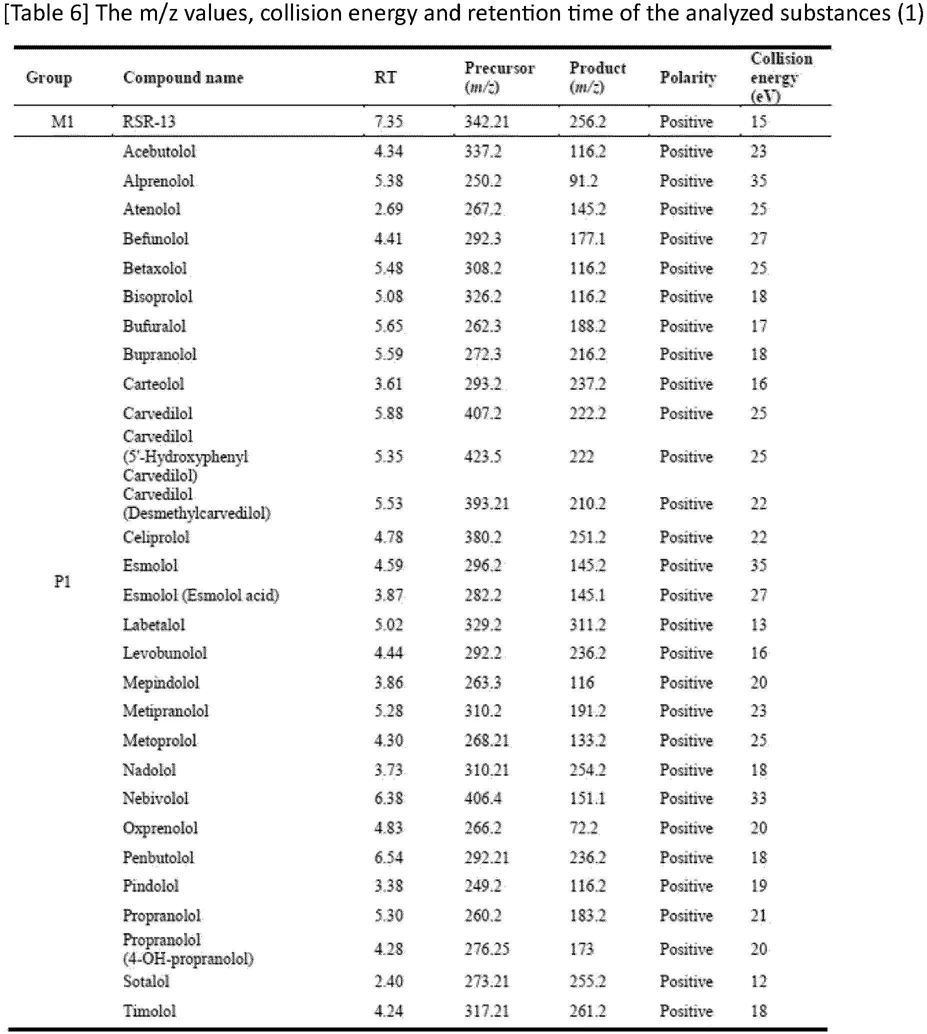

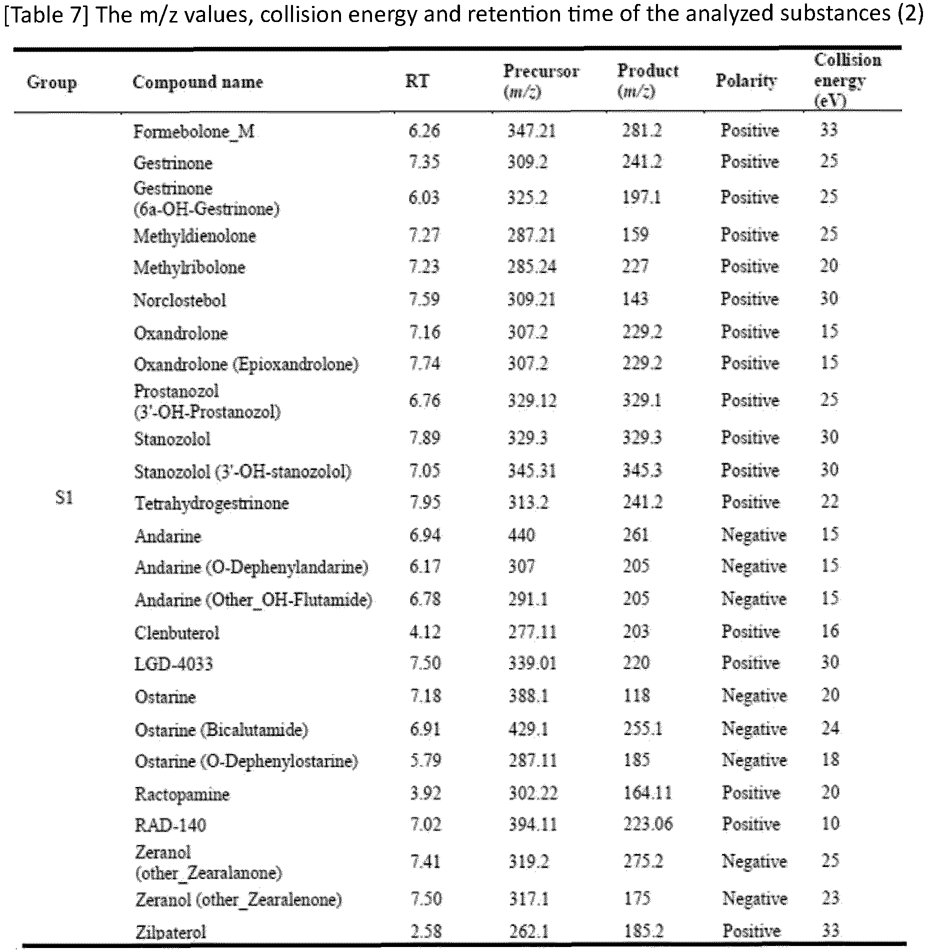

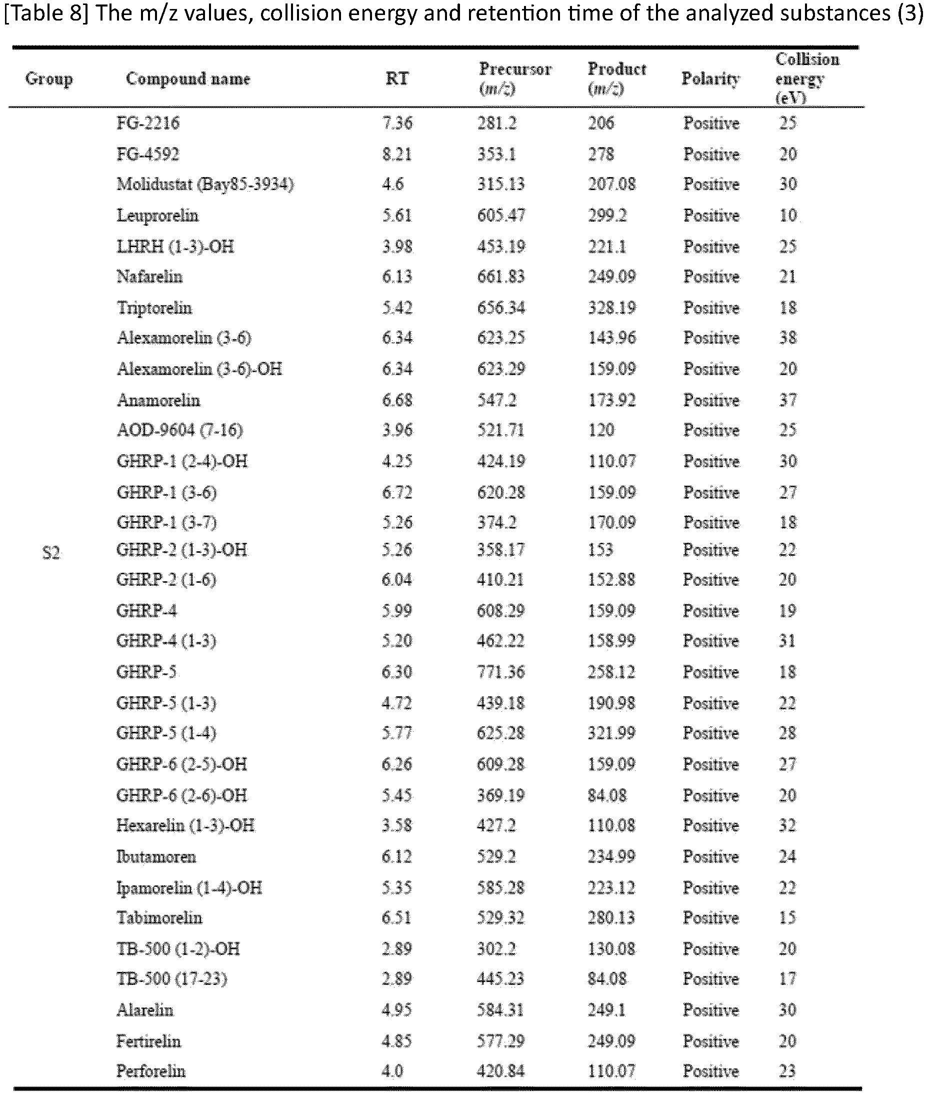

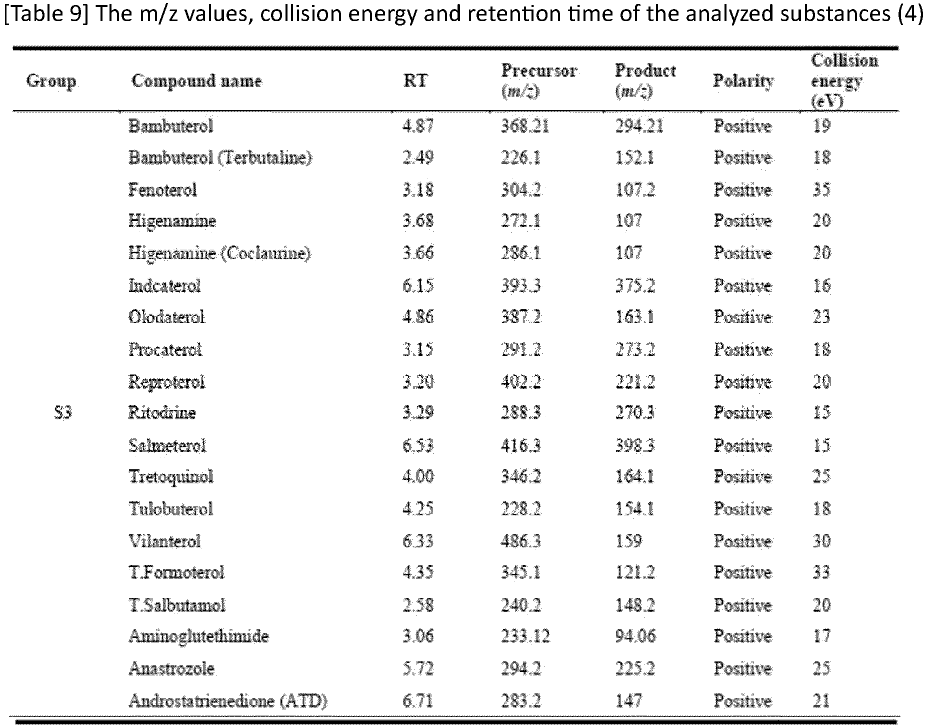

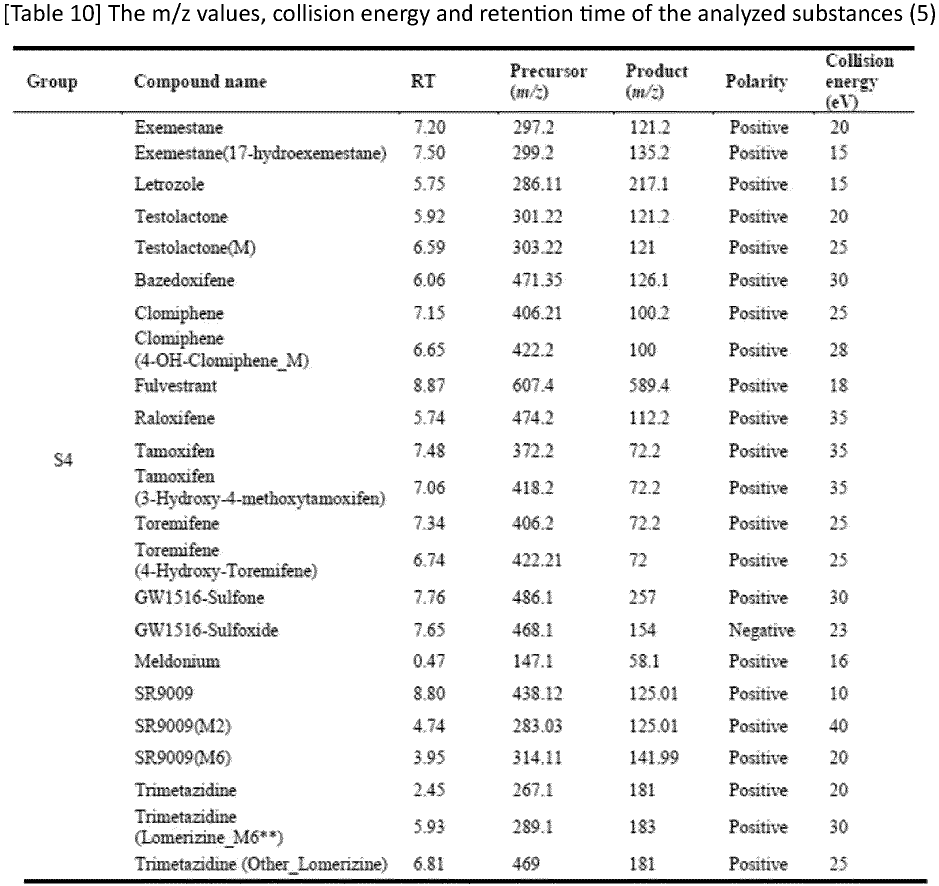

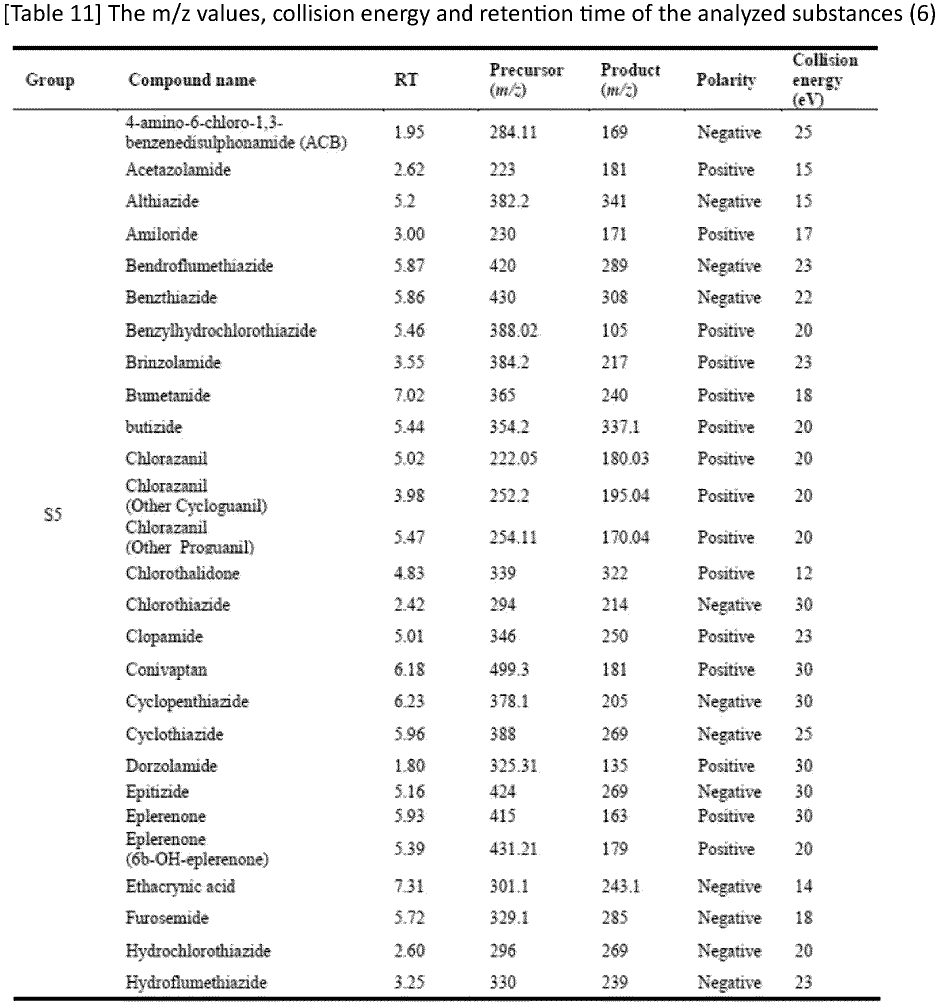

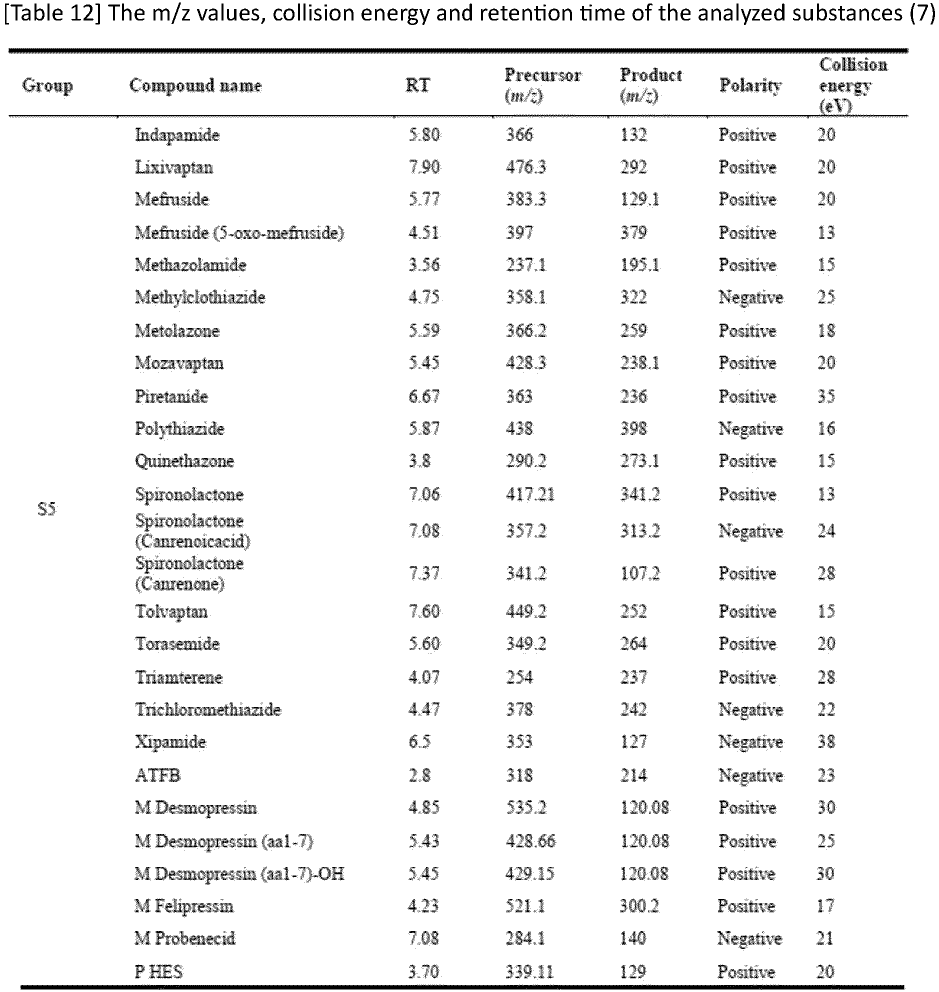

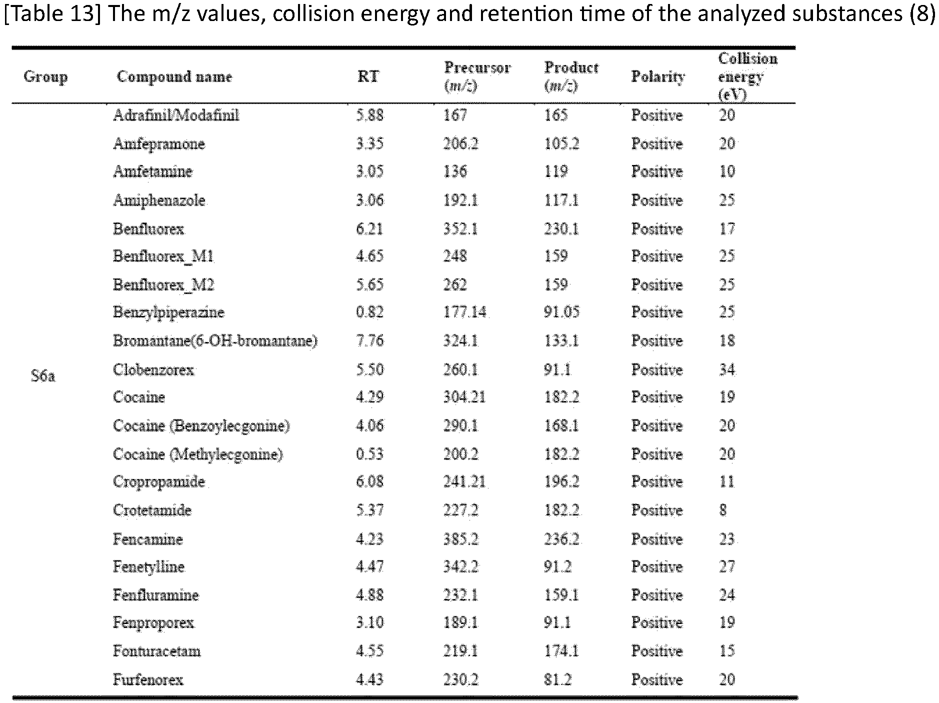

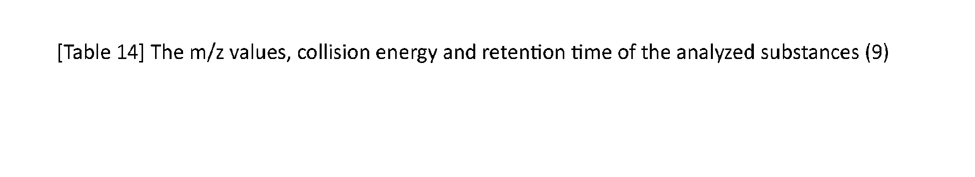

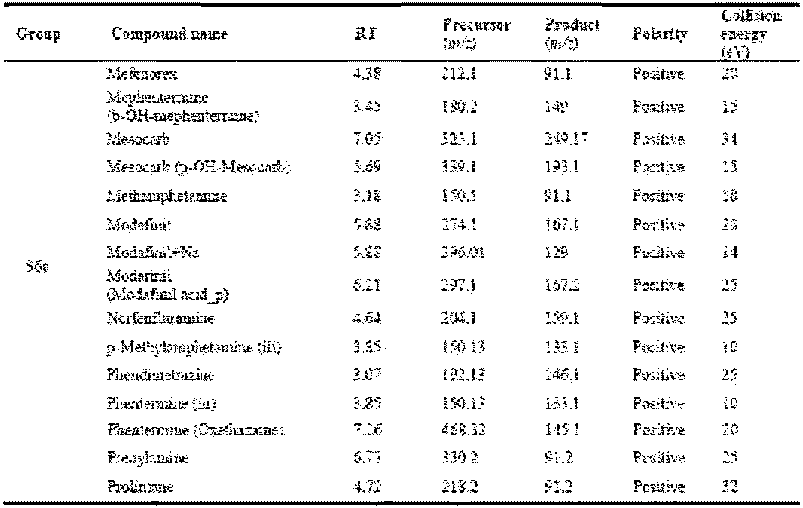

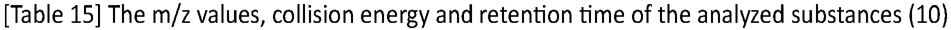

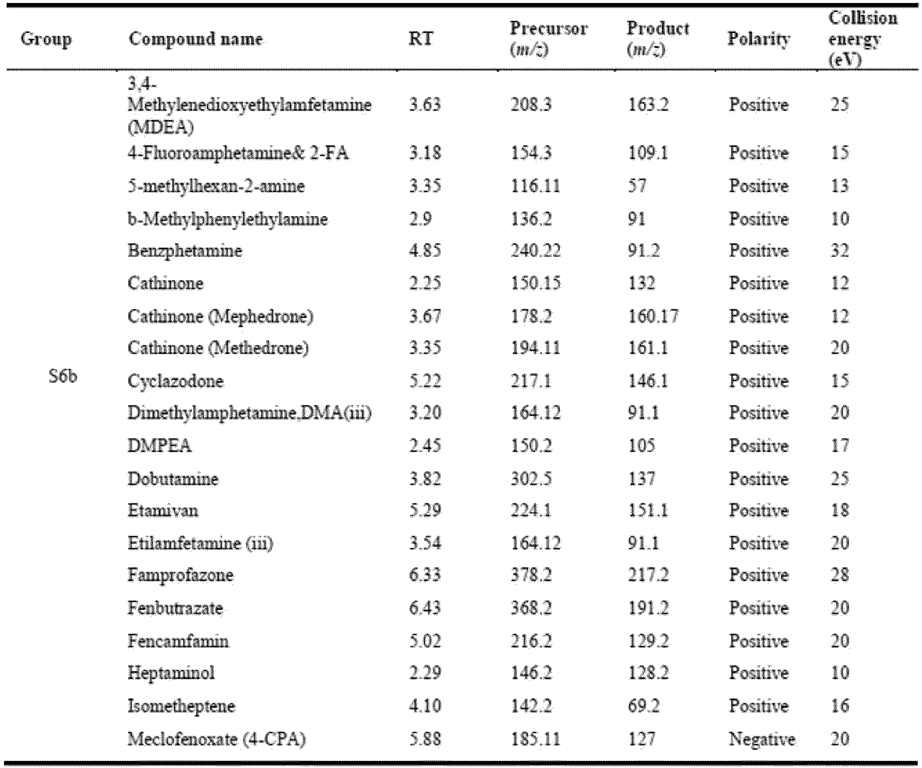

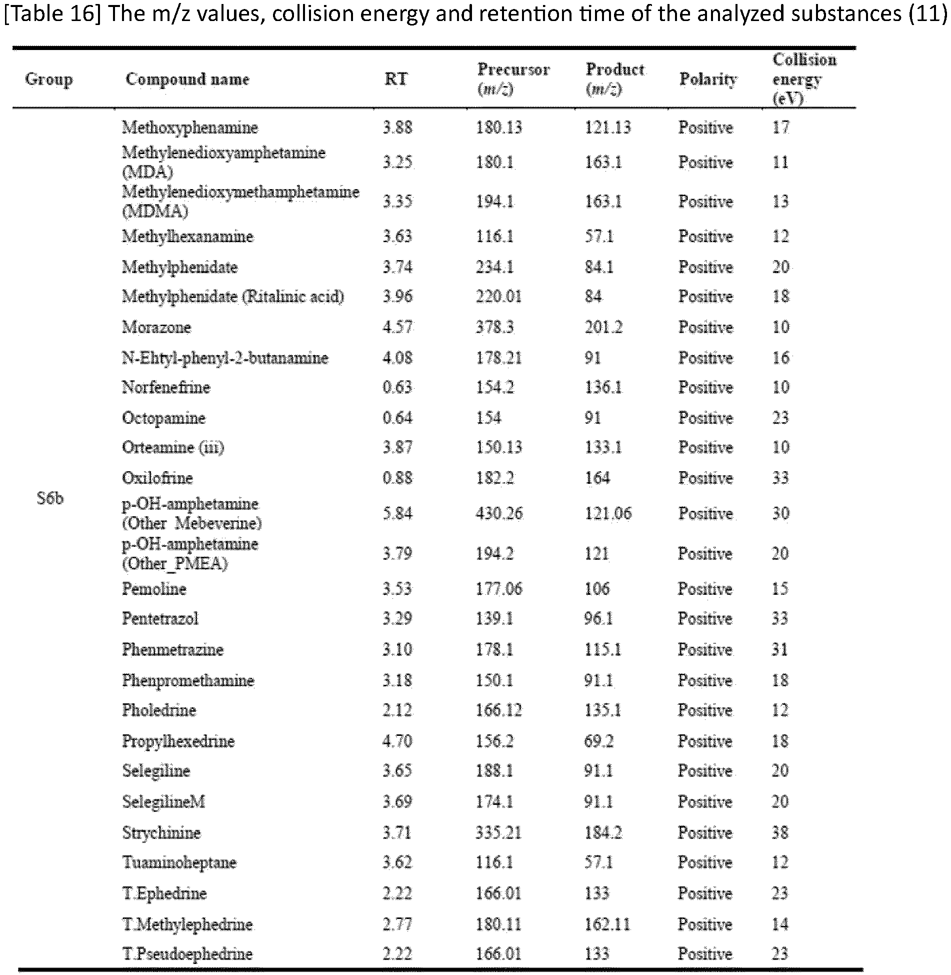

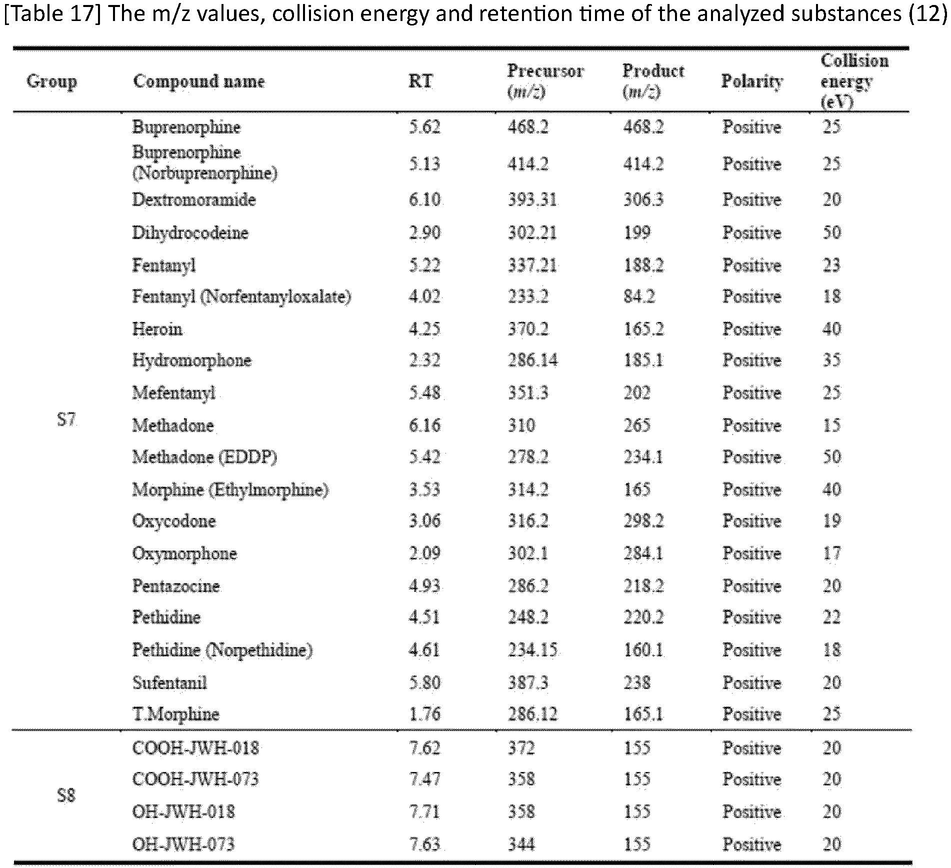

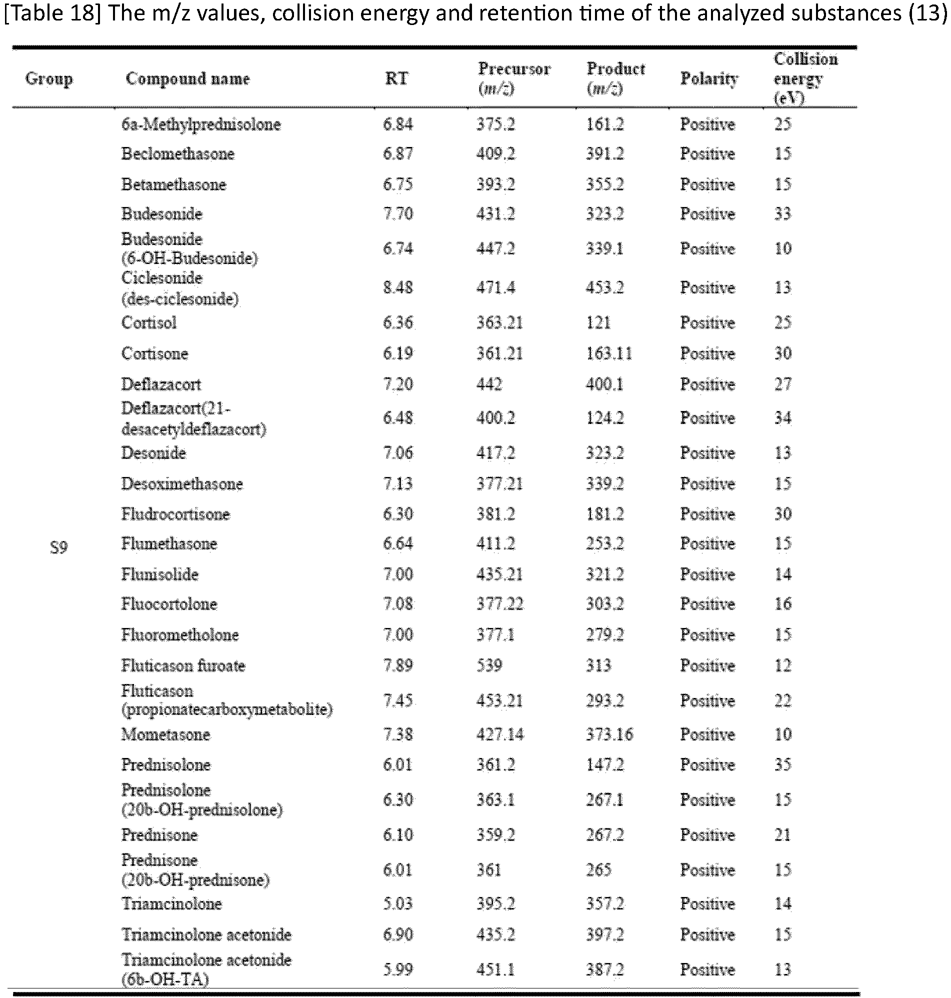

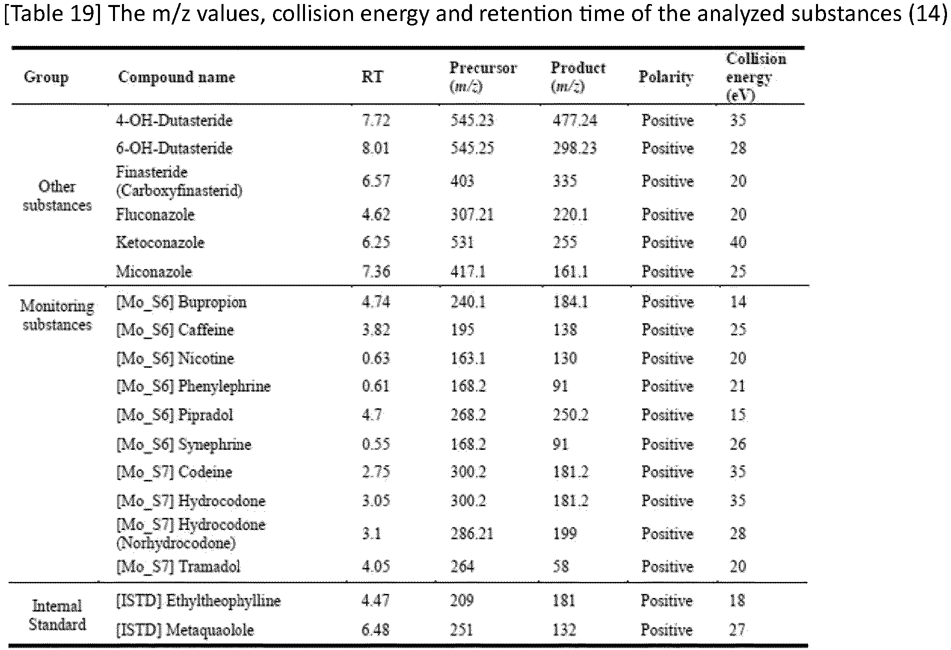

As shown in Table 6 to Table 19, it was confirmed that the simultaneous analysis method of food-borne doping substances using the Quechers sample pretreatment method and liquid chromatography-mass spectrometry/mass spectrometry (LC-MS/MS) of the present invention can be used to quickly and accurately detect multiple doping substances in the food matrix simultaneously.

## Claims

1. A method for simultaneous analysis of food-borne doping substances comprises the steps of:
(a) homogenizing food sample and then preparing an analytical sample by mixing it with water and acetonitrile;
(b) pretreating the analytical sample with a salt mixture comprising MgSO₄, NaCl, Na₃C₆H₅O₇, and C₆H₆Na₂O₇-1.5H₂O;
(c) purifying the pretreated analytical sample by QuEChERS dispersive solid phase extraction method (dSPE) using MgSO₄, C18EC column, graphitized carbon black (GCB) and Primary Secondary Amine (PSA; N-propyl ethylene diamine based SPE column);
(d) concentrating the analytical sample with a nitrogen concentrator and then redissolving the analytical sample in a 0.1% (v/v) formic acid aqueous solution; and
(e) centrifuging the redissolved analytical sample and then analyzing the supernatant by liquid chromatography-mass spectrometry (LC-MS/MS) for simultaneous analysis of food-borne doping substances.

2. The method for simultaneous analysis of food-borne doping substances of claim 1, wherein the liquid chromatography-mass spectrometry of step (e) uses a Kinetex 2.6-µm C18 column and a Security Guard ULTRA Cartridges column.

3. The method for simultaneous analysis of food-borne doping substances of claim 1, wherein the liquid chromatography-mass spectrometry of step (e) is performed using water with 0.1% (v/v) formic acid (solution A) and methanol with 0.1% (v/v) formic acid (solution B) as the mobile phase, and is performed using a gradient of 2% (v/v) B solution (0 to 0.5 min); 2% (v/v) to 95% (v/v) B solution (0.5 to 8.5 min); 95% (v/v) B solution (8.5 to 9 min); 95% (v/v) to 2% (v/v) B solution (9 to 9.01 min); and 2% (v/v) B solution (9.01 to 10 min).

4. The method for simultaneous analysis of food-borne doping substances of claim 1, wherein the mobile phase is injected into liquid chromatography at a rate of 0.4 to 0.6 mL/min.

5. The method for simultaneous analysis of food-borne doping substances of claim 1, wherein the food is an agricultural product, livestock product, natural spice, dairy product, processed animal product, edible oil, beverage, processed food, ready-to-eat food, snack, or dietary supplement.

6. The method for simultaneous analysis of food-borne doping substances of claim 1, wherein the doping substances detected simultaneously by the method are at least one selected from the group consisting of: Efaproxiral (RSR-13), Acebutolol, Alprenolol, Atenolol, Befunolol, Betaxolol, Bisoprolol, Bufuralol, Bupranolol, Carteolol, Carvedilol, Carvedilol(5'-Hydroxyphenyl Carvedilol), Carvedilol(Desmethylcarvedilol), Celiprolol, Esmolol, Esmolol acid, Labetalol, Levobunolol, Mepindolol, Metipranolol, Metoprolol, Nadolol, Nebivolol, Oxprenolol, Penbutolol, Pindolol, Propranolol, Propranolol(4-OH-propranolol), Sotalol, Timolol, Formebolone_M, Gestrinone, Gestrinone (6a-OH-Gestrinone), Methyldienolone, Methylribolone, Norclostebol, Oxandrolone, Oxandrolone(Epioxandrolone), Prostanozol (3'-OH-Prostanozol), Stanozolol, Stanozolol(3'-OH-stanozolol)], Tetrahydrogestrinone, Andarine, Andarine(O-Dephenylandarine), Andarine(Other_OH-Flutamide), Clenbuterol, LGD-4033, Ostarine, Ostarine(Bicalutamide), Ostarine(O-Dephenylostarine), Ractopamine, RAD-140, Zeranol(other_Zearalanone), Zeranol(other_Zearalenone), Zilpaterol, FG-2216(HIF-prolyl hydroxylase inhibitor), FG-4592(Prolyl hydroxylase inhibitor), Molidustat (Bay85-3934), Leuprorelin, LHRH(1-3)-OH([luteinizing hormone(LH)-releasing hormone (1-3)-OH], Nafarelin, Triptorelin, Alexamorelin (3-6), Alexamorelin (3-6)-OH, Anamorelin, AOD-9604(7-16), GHRP(growth hormone-releasing peptide)-1 (2-4)-OH, GHRP-1(3-6), GHRP-1(3-7), GHRP-2(1-3)-OH, GHRP-2(1-6), GHRP-4, GHRP-4(1-3), GHRP-5, GHRP-5(1-3), GHRP-5(1-4), GHRP-6(2-5)-OH, GHRP-6(2-6)-OH, Hexarelin (1-3)-OH, Ibutamoren, Ipamorelin (1-4)-OH, Tabimorelin, TB-500(1-2)-OH[Thymosin Beta-4(1-2)-OH], TB-500(17-23), Alarelin, Fertirelin, Perforelin, Bambuterol, Bambuterol(Terbutaline), Fenoterol, Higenamine, Higenamine (Coclaurine), Indcaterol, Olodaterol, Procaterol, Reproterol, Ritodrine, Salmeterol, Tretoquinol, Tulobuterol, Vilanterol, T.Formoterol, T.Salbutamol, Aminoglutethimide, Anastrozole, Androstatrienedione (ATD), Exemestane, Exemestane(17-hydroexemestane), Letrozole, Testolactone, Testolactone M, Bazedoxifene, Clomiphene(4-OH-Clomiphene_M), Fulvestrant, Raloxifene, Tamoxifen, Tamoxifen(3-Hydroxy-4-methoxytamoxifen), Toremifene, Toremifene(4-Hydroxy-Toremifene), GW1516-Sulfone, GW1516-Sulfoxide, Meldonium, SR9009, SR9009(M2), SR9009(M6), Trimetazidine, Trimetazidine(Lomerizine_M6), Trimetazidine(Other_Lomerizine), 4-amino-6-chloro-1,3-benzenedisulphonamide (ACB), Acetazolamide, Althiazide, Amiloride, Bendroflumethiazide, Benzthiazide, Benzylhydrochlorothiazide, Brinzolamide, Bumetanide, Butizide, Chlorazanil, Chlorazanil(Other Cycloguanil), Chlorazanil(Other_Proguanil), Chlorothalidone, Chlorothiazide, Clopamide, Conivaptan, Cyclopenthiazide, Cyclothiazide, Dorzolamide, Epitizide, Eplerenone, Eplerenone(6b-OH-eplerenone), Ethacrynic acid, Furosemide, Hydrochlorothiazide, Hydroflumethiazide, Indapamide, Lixivaptan, Mefruside, Mefruside(5-oxo-mefruside), Methazolamide, Methylclothiazide, Metolazone, Mozavaptan, Piretanide, Polythiazide, Quinethazone, Spironolactone, Spironolactone(Canrenoicacid), Spironolactone(Canrenone), Tolvaptan, Torasemide, Triamterene, Trichloromethiazide, Xipamide, ATFB, M Desmopressin, M Desmopressin(aa1-7], M Desmopressin(aal-7)-OH, M Felipressin, M Probenecid, P HES(hydroxyethyl starche), Adrafinil/Modafinil, Amfepramone, Amfetamine, Amiphenazole, Benfluorex, Benfluorex_M1, Benfluorex_M2, Benzylpiperazine, Bromantane(6-OH-bromantane), Clobenzorex, Cocaine, Cocaine (Benzoylecgonine), Cocaine (Methylecgonine), Cropropamide, Crotetamide, Fencamine, Fenetylline, Fenfluramine, Fenproporex, Fonturacetam, Furfenorex, Mefenorex, Mephentermine (b-OH-mephentermine), Mesocarb, Mesocarb (p-OH-Mesocarb), Methamphetamine, Modafinil, Modafinil+Na, Modarinil(Modafinil acid_p), Norfenfluramine, p-Methylamphetamine (iii), Phendimetrazine, Phentermine (iii), Phentermine (Oxethazaine), Prenylamine, Prolintane, 3,4-Methylenedioxyethylamfetamine (MDEA), 4-Fluoroamphetamine& 2-FA), 5-methylhexan-2-amine, b-Methylphenylethylamine, Benzphetamine, Cathinone, Cathinone (Mephedrone), Cathinone (Methedrone), Cyclazodone, Dimethylamphetamine, DMA(iii), 3,4-Dimethoxyphenethylamine (DMPEA), Dobutamine, Etamivan, Etilamfetamine (iii), Famprofazone, Fenbutrazate, Fencamfamin, Heptaminol, Isometheptene, Meclofenoxate(4-CPA), Methoxyphenamine, Methylenedioxyamphetamine (MDA), Methylenedioxymethamphetamine (MDMA), Methylhexanamine, Methylphenidate, Methylphenidate (Ritalinic acid), Morazone, N-Ehtyl-phenyl-2-butanamine, Norfenefrine, Octopamine, Orteamine (iii), Oxilofrine, p-OH-amphetamine (Other_Mebeverine), p-OH-amphetamine (Other_PMEA)], Pemoline, Pentetrazol, Phenmetrazine, Phenpromethamine, Pholedrine, Propylhexedrine, Selegiline, Selegiline M, Strychinine, Tuaminoheptane, T.Ephedrine, T.Methylephedrine, T.Pseudoephedrine, Buprenorphine, Buprenorphine(Norbuprenorphine), Dextromoramide, Dihydrocodeine, Fentanyl, Fentanyl(Norfentanyloxalate), Heroin, Hydromorphone, Mefentanyl, Methadone, Methadone(EDDP), Morphine(Ethylmorphine), Oxycodone, Oxymorphone, Pentazocine, Pethidine, Pethidine(Norpethidine), Sufentanil, T.Morphine, COOH-JWH-018, COOH-JWH-073, OH-JWH-018, OH-JWH-073, 6a-Methylprednisolone, Beclomethasone, Betamethasone, Budesonide, Budesonide(6-OH-Budesonide], Ciclesonide(des-ciclesonide), Cortisol, Cortisone, Deflazacort, Deflazacort(21-desacetyldeflazacort), Desonide, Desoximethasone, Fludrocortisone, Flumethasone, Flunisolide, Fluocortolone, Fluorometholone, Fluticason furoate, Fluticason(propionatecarboxymetabolite), Mometasone, Prednisolone, Prednisolone(20b-OH-prednisolone), Prednisone, Prednisone(20b-OH-prednisone), Triamcinolone, Triamcinolone acetonide, Triamcinolone acetonide(6b-OH-TA)], 4-OH-Dutasteride, 6-OH-Dutasteride, Finasteride (Carboxyfinasteride), Fluconazole, Ketoconazole, Miconazole, Bupropion, Caffeine, Nicotine, Phenylephrine, Pipradol, Synephrine, Hydrocodone, Hydrocodone(Norhydrocodone), and Tramadol.
